# EUROPEAN PATENT SPECIFICATION

(11) **EP 1 206 542 B1**
(45) Date of publication and mention of the grant of the patent: **09.11.2005**
(21) Application number: 00954207.7
(22) Date of filing: 17.08.2000
(51) Int. Cl.: C12N 15/12, C12N 15/62, C07K 14/47, A61K 48/00

(54) **REPLICATION PROTEIN A BINDING TRANSCRIPTIONAL FACTOR (RBT1) AND USES THEREOF**
REPLIKATIONSPROTEIN-A-BINDENDES TRANSKRIPTIONSFAKTOR (RBT1) UND DESSEN VERWENDUNGEN
FACTEUR TRANSCRIPTIONNEL DE LIAISON DE PROTEINE DE REPLICATION A (RBT1) ET SES UTILISATIONS

(30) Priority: 19.08.1999 US 149472 P
(43) Date of publication of application: 22.05.2002
(73) Proprietor: Centre for Translational Research in Cancer, Montreal, Québec H3T 1E2 (CA)
(72) Inventor: ALAOUI-JAMALI, Moulay, A., Laval, Québec H7G 2P1 (CA); CHO, John Myung-Jae, Westmount, Québec H3Z 1H2 (CA)
(74) Representative: Robertson, James Alexander
(86) International application number: PCT/CA2000/000948
(87) International publication number: WO 2001/014546

(56) References cited:
- WO-A-98/00013
- WO-A-98/37901
- DATABASE EM_EST [Online] EMBL; ID HS1272698, AC AA482412, 24 June 1997 (1997-06-24) HILLIER L ET AL.: "zt34d02.r1 Soares ovary tumor NbHOT Homo sapiens cDNA clone IMAGE:724227 5', mRNA sequence" XP002167205
- DATABASE EM_EST [Online] EMBL; ID AA690874, AC AA690874, 19 December 1997 (1997-12-19) MARRA M ET AL.: "vt32d01.r1 Barstead mouse proximal colon MPLRB6 Mus musculus cDNA clone IMAGE:1164769 5', mRNA sequence" XP002167206
- NAGELHUS TA ET AL.: "A sequence in the N-terminal region of human uracil-DNA glycosylase with homology to XPA interacts with the C-terminal part of the 34-kDa subunit of replication protein A" JOURNAL OF BIOLOGICAL CHEMISTRY, vol. 272, no. 10, 7 March 1997 (1997-03-07), pages 6561-6566, XP002167201
- IFTODE C ET AL.: "Replication protein A (RPA): the eukaryotic SSB" CRITICAL REVIEWS IN BIOCHEMISTRY AND MOLECULAR BIOLOGY, vol. 34, no. 3, 1999, pages 141-180, XP001002021
- WOLD MS: "Replication protein A: a heterotrimeric, single-stranded DNA-binding protein required for eukaryotic DNA metabolism" ANNUAL REVIEW OF BIOCHEMISTRY, vol. 66, 1997, pages 61-92, XP001002023 cited in the application
- DATABASE EM_HUM [Online] EMBL; ID AC010271, AC AC010271, 17 September 1999 (1999-09-17) DOE JOINT GENOME INSTITUTE STANFORD HUMAN GENOME CENTER: "Homo sapines chromosome 19 CTC-492K19, complete sequence" XP002167207
- CHO J M ET AL.: "RBT1, a novel transcriptional co-activator, binds the second subunit of Replication Protein A" NUCLEIC ACIDS RESEARCH, vol. 28, no. 18, 15 September 2000 (2000-09-15), pages 3478-3485, XP002167204

## Description

### BACKGROUND OF THE INVENTION

### (a) Field of the Invention

The invention relates to Replication Protein A (RPA) and more particularly to a RPA transcriptional factor.

### (b) Description of Prior Art

Replication Protein A (RPA), also known as replication factor A (RFA), is a ubiquitous and abundant heterotrimeric protein required for DNA replication, repair and recombination in eukaryotes. RPA nonspecifically binds single-stranded DNA and plays an essential role in the regulation of DNA metabolism *via* multiple protein interactions and/or RPA phosphorylation. More particularly, RPA binds single-stranded DNA with strong affinity (association constant of 10⁹-10¹¹ M⁻¹) and greatest affinity for polypyrimidine tracts. RPA also binds double-stranded DNA with lower affinity and is likely to facilitate DNA unwinding. RPA may play a role in the regulation of transcription by binding regulatory elements in promoters; in yeast, RPA binds specific regulatory sequences in the promoters of DNA repair and metabolism genes (Singh K. *et al*., 1995, *Proceedings of the National Academy of Science USA* **92** (11) :4907-11).

RPA is made of three subunits: a 70-kDa subunit (RPA70), a 32-kDa middle subunit (RPA32) and a 14-kDa subunit (RPA14). The RPA32 subunit is phosphorylated in a cell cycle-dependent manner.

RPA-protein interactions appear to be largely mediated by the large 70-kDa subunit (RPA70). RPA70 interacts with the p53, GAL4, VP16, EBNA1 and SV40T antigens and with DNA polymerase alpha (Wold, M., 1997, *Annual Review of Biochemistry,* "Replication Protein A: A Heterotrimeric, Single-Strahded DNA-binding Protein Required for Eukaryotic DNA Metabolism"). It is also important in interaction with DNA repair proteins involved in damage recognition and excision.

Interaction with XPF stimulates its 5' junction-specific endonuclease activity, interaction with XPG targets this endonuclease to damaged DNA, and interaction with ERCC1 (ERCC1 also binds *xeroderma pigmentosum* group A factor, XPA, which is another NER factor) promotes exonuclease activity.

The possibility of interaction by the aforementioned repair proteins with RPA32 has not been clearly elucidated. However, interactions with some proteins involved in DNA repair appear to be mediated by RPA32, such as interaction with XPA and uracil-DNA glycosylase (Nagelhus T.A. et al., 1997, Journal of Biological Chemistry, 272 (10), p6561-6566). A region of significant homology between uracil-DNA glycosylase and XPA was also reported, suggestive of the possibility of a common binding motif to RPA32 across several different proteins. Furthermore, some important protein interactions, such as with RAD52, appear to involve all three subunits of RPA (Hays, S. *et al*., 1998, *Molecular and Cellular Biology* **18** (7) :4400-4406).

In cells, RPA is phosphorylated by DNA-dependent protein kinase (DNA-PK) when RPA is bound to single-strand DNA, during the S phase and after DNA damage; and also possibly by ATM.

Phosphorylation of RPA is observed in a cell-cycle dependent manner and in response to DNA damage (i.e. UV light, X-rays, cisplatin) in eukaryotic systems. This phosphorylation takes place predominantly on the N-terminal region of RPA32 and was previously thought to be effected by DNA-dependent protein kinase (DNA-PK). However, RPA hyperphosphorylation still takes place in SCID cells where DNA-PK is believed to be responsible for its repair and recombination defects. Ataxia telangiectasia mutated gene (ATM), an important cell cycle checkpoint protein kinase belonging to the same kinase family as DNA-PK, may be responsible for the *in vivo* phosphorylation of RPA32. In *Saccharomyces cerevisiae*, the ATM homolog, MEC1, is essential for RPA phosphorylation. Furthermore, ionizing radiation-induced phosphorylation of RPA32 is deficient and reduced in primary fibroblasts from patients suffering from ataxia telangiectasia in comparison to normal, aged fibroblasts.

The result of RPA32 phosphorylation on DNA metabolism is largely unsolved. It has been noted that IR-induced RPA phosphorylation can be uncoupled from the S-phase checkpoint in ataxia telangiectasia cells, suggesting that RPA phosphorylation in itself is not necessary or sufficient for an S-phase arrest. Phosphorylation, however, may affect the conformation of RPA, thereby modulating its affinity for DNA and its protein interactors, and altering the balance between DNA replication and repair. Hyperphosphorylation of RPA32 *in vivo* is concordant with a decrease in the binding of RPA to single-stranded DNA. This observation is interesting to note since phosphorylated RPA32 is found predominantly in the S-phase of the cell cycle.

RPA has been found to have a high affinity for UV-damaged and cisplatin-damaged DNA and the accompanying phosphorylated form of RPA is correlated strongly with a reduction of the *in vitro* DNA replication activity of the concerned cell extracts.

It would therefore be highly desirable to identify physiologically relevant protein interactors of the RPA32 subunit of Replication Protein A. Identification of such protein interactors would contribute to the understanding of DNA repair, transcription, and cell signaling. The proteins involved in nucleotide excision repair (NER), for example, are quite numerous and the basis for their interaction and function is not yet completely understood. Understanding the regulation of these pathways would assuredly lend insight into their role in cancer susceptibility. RPA, as a protein involved integrally in modulating DNA repair, replication and recombination, would be key to understanding the connection between and within pathways. The implications to cancer therapeutics and/or prevention would be significant.

### SUMMARY OF THE INVENTION

One aim of the present invention is to provide a protein interactor of the RPA32 subunit of Replication Protein A (RPA).

In accordance with the present invention, there is provided a gene encoding a protein consisting in the amino acid sequence as set forth in SEQ ID NO:2.

The gene may be from a human, a mouse, a rat or a yeast.

In accordance with the present invention, there is also provided a protein consisting in the amino acid sequence set forth in SEQ ID NO:2.

The protein may be from a human, a mouse, a rat or a yeast.

Antibodies may be raised against the gene.

The gene, replication protein A binding transcriptional activator 1 (RBT1), encodes a protein interactor of the Replication Protein A (RPA). More particularly, a protein interactor of the Replication Protein A 32KD subunit was identified. RBT1 binds RPA32.

The RBT1 gene has a high activity in cancer cells compared to normal cells, may be involved in carcinogenesis and is highly transactivated in cancer cells.

The RBT1 nucleotide and/or amino acid sequences may be used to generate reagents, such as plasmids and antibodies.

### BRIEF DESCRIPTION OF THE DRAWING

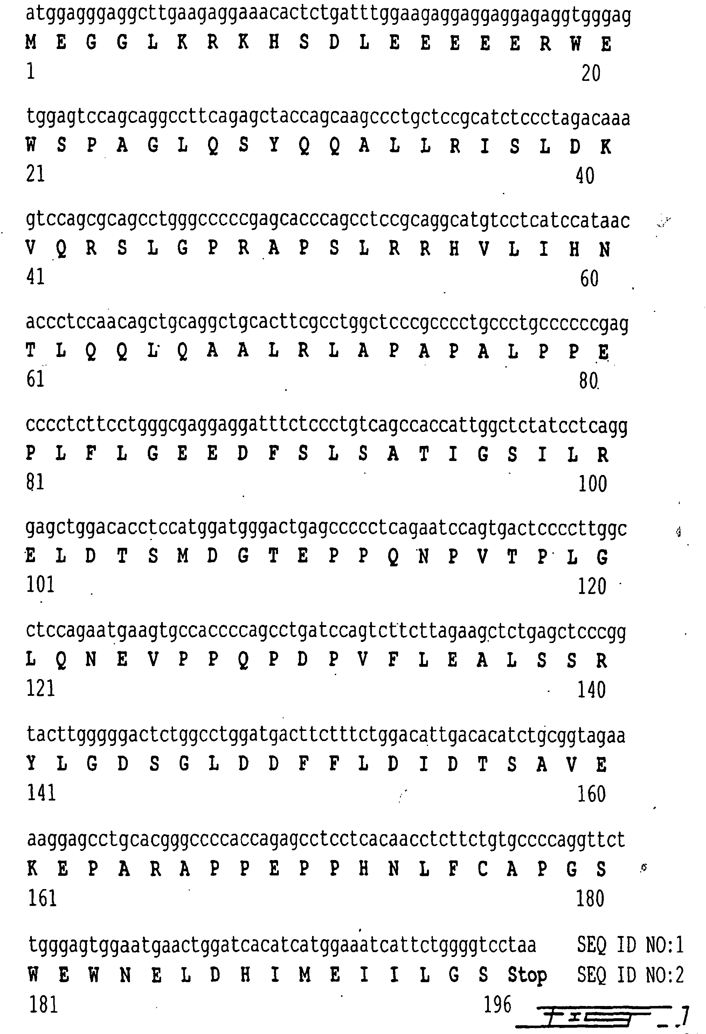

Fig. 1 illustrates the nucleotide (SEQ ID NO:1) and the amino acid sequence (SEQ ID NO:2) of RBT1.

### DETAILED DESCRIPTION OF THE INVENTION

In accordance with the present invention, there is provided a gene sequence encoding a protein interactor of Replication Protein A, identified using the yeast two-hybrid system. The gene, named RPA Binding Transcriptional Activator 1 (RBT1), has a putative open reading frame of 196 amino acids. The coding sequence of RBT1 corresponds to several expressed sequence tags (ESTs), including one derived from an ovary tumor cell line. The gene of the present invention acts as a strong transcriptional activator in yeast and mammalian cells. Furthermore, transcriptional activation, as assayed by a luciferase reporter gene, demonstrated that the activity of the RBT1 gene of the present invention is higher in cancer cells compared to normal non-immortalized cells. RBT1 expression is higher in cancer cells compared to normal cells. More particularly, a protein interactor of Human Replication Protein A 32 (RPA32) was identified.

BLASTP homology searches against the deduced amino acid sequence of RBT1 reveal that it is an undefined protein with little homology to known protein sequences. Further, BLASTN homology searches only identified approximately 20 human expressed sequence tags (ESTs) which had high homology to RBT1.

Northern blot using an RBT1 DNA probe showed one transcript of approximately 1.55 kb in size. *In silicio* analysis suggested that RBT1 consists of an open reading frame (ORF) of 196 amino acids and a theorectical molecular weight of 22 kDa. This is in agreement with Western Blot analysis.

Differential expression of RBT1 was also investigated as it relates to cancer. Semi-quantitative analysis has shown that RBT1 is at least ten times more expressed in cell line H661 (cancer cells) than NHBEC (normal cells).

Various cell lines are investigated to ascertain whether RBT1 has relevance to carcinogenesis. *In silicio* analysis also suggests that the N-terminal domain of RBT1 contains a putative DNA binding domain. Whether RBT1 binds specific DNA regulatory elements is also being investigated.

The presence of an acidic domain in the C-terminal domain of RBT1 led to investigate whether RBT1 was a potential transcriptional activator. RBT1, fused to the LexA binding domain, strongly promotes transcription of reporter genes LacZ and HIS3 in the yeast two-hybrid system, suggesting its possible role as a transcriptional activator.

RBT1 deletion constructs were designed to determine the transactivating domain, and to define the domain which is essential for RPA32 interaction. The transactivation domain of RBT1 resides within 30 amino acids at the C-terminal. Truncation of RBT1 from the C-terminal end results in significant reduction of transactivation of the reporter genes.

A mammalian transactivation assay confirmed that a GAL4-RBT1 fusion protein indeed acts as a strong transcriptional activator. Furthermore, transcriptional activation, as assayed by a luciferase reporter gene, although high in all cancer cell lines examined, is at least 4 times higher in cell line MCF7. Transactivation studies were also performed using a mammalian system to verify that RBT1 acts as a transcriptional activator in its native cellular environment. RBT1, fused to a GAL4 DNA binding domain, strongly promotes transcription of the luciferase reporter gene. This transactivation was strongly attenuated by truncation of the gene from the C-terminal end. Further, the relative transactivation of the luciferase reporter by the full length RBT1 gave values higher than the positive control construct, TLS, in cell lines 293, MDA231, MCF7 and Saos-2, suggesting a possible role in cancer.

RBT1 binds RPA32 in the yeast two-hybrid system. Truncation of RBT1 suggests that the domain of RBT1 responsible for binding to RPA32 resides between amino acid 1-120. This region also contains a putative DNA binding domain which needs to be clarified. A GST-RBT1 construct was found to bind *in vitro* translated RPA32. A GFP-RBT1 was transfected into cell line 293 and shown to localize in the nucleus in a pattern similar to, and possibly overlapping with, that of RPA32.

RPA32 was amplified from MCF7 cDNA and cloned, in frame, into pBTM116, a LexA two-hybrid plasmid subsequently referred to as RPA34-pBTM116. This plasmid construct was transformed into yeast strain L40 (MATa trp1 leu2 his3 URA3:(lexAop)8-lacZ LYS2::(lexAop)4-HIS3 lys2 ura3 ade2 gal80 gal4) prior to library transformation. Transcription of the HIS3 reporter gene was found to occur in the absence of protein-protein interaction; this was attributed to potential transactivation function of RPA32. To reduce background, 3-aminotriazole (3-AT), a metabolic inhibitor, was included in media lacking histidine to increase the stringency of the screen. A human osteosarcoma GAL4 cDNA library was amplified according to CLONTECH recommended protocols.

400,000 colonies were screened and 72 colonies were identified which were able to grow on media lacking histidine and containing 25 mM 3-AT. These colonies were restreaked on the same media and replicated to media containing X-gal. Based on growth rates on SC-histidine (25 mM 3-AT) media and on level of induction of the B-gal reporter gene, positive colonies were classified into three groups, strong, intermediate, and weak interactors. All colonies which demonstrated high levels of induction of the B-gal reporter gene were assayed by PCR using a primer specific to RPA14 and ADC1, a sequence in the terminator region of the library plasmid. Interacting plasmids from several of these colonies were purified and sequenced. Both PCR and sequencing experiments showed that the strongest interactors were representative of RPA32.

A putative positive identified as a RPA32 interacting protein was sequenced and found to have no strong homology to known proteins. This gene, referred to as RBT1, was subcloned from the pACT2 vector into pBTM116 for purposes of mini-screening the two-hybrid library. However, the library could not be screened using RBT1-pBTM116 as bait because of extremely strong transactivation of the yeast reporter genes by itself. This observation suggests that RBT1 may be a transcriptional activator. Although some proteins fortuitously show transcriptional transactivation, its binding to RPA32 supports the notion that RBT1 may not be among such proteins. Experiments were done in attempts to ascertain whether RBT1 functions as a transcriptional activator.

There are several dbEST matches for RBT1 in GenBank. Two representative full length clones have been obtained, DNA was purified, and may be sequenced completely.

Plasmid constructs with truncations at the 3' end of RBT1 have been cloned and transformed into yeast strain L40. ONPG assays shows substantial diminishment of B-gal activity with just 60 bp deleted from the 3', suggesting that the potential transcriptional activation domain of RBT1 lies at the carboxy terminal.

Similar constructs may be cloned into a vector for transfection into human cells, using an in-frame fusion to GAL4 DNA-binding domain and utilizing a second plasmid bearing a luciferase reporter gene under the control of several GAL4 binding sites. These experiments determine whether the transactivation found in the yeast system are physiologicallly relevant.

RBT1 may be overexpressed in various human cell lines to ascertain possible phenotypic effects. Experiments may include UV and chemical challenge.

Antibodies against RBT1 may be raised for subsequent protein localization experiments in human cells. This antibody may also be used for various co-immunoprecipitation experiments to show RPA-RBT1 binding.

## Claims

1. A gene encoding a protein consisting in the amino acid sequence as set forth in SEQ ID NO:2.

2. A gene according to claim 1 said gene being from a species selected from the group consisting of human, mouse, rat and yeast.

3. A protein consisting in the amino acid sequence set forth in SEQ ID NO:2.

4. A protein according to claim 3, said protein being from a species selected from the group consisting of human, mouse, rat and yeast.

5. An antibody raised against a gene according to claim 1.

6. An antibody raised against a protein according to claim 3.

## Patentansprüche

1. Gen, codierend für ein Protein, das in der wie in SEQ ID NO:2 dargelegten Aminosäuresequenz besteht.

2. Gen nach Anspruch 1, wobei das Gen von einer Spezies stammt, die aus der Gruppe ausgewählt ist, bestehend aus einem Menschen, einer Maus, einer Ratte und einer Hefe.

3. Protein, bestehend in der in SEQ ID NO:2 dargelegten Aminosäuresequenz.

4. Protein, nach Anspruch 3, wobei das Protein von einer Spezies stammt, die aus der Gruppe ausgewählt ist, bestehend aus einem Menschen, einer Maus, einer Ratte und einer Hefe.

5. Antikörper, gebildet gegen ein Gen nach Anspruch 1.

6. Antikörper, gebildet gegen ein Protein nach Anspruch 3.

## Revendications

1. Un gène codant une protéine dont la séquence d'acides aminés consiste en la séquence décrite dans SEQ ID NO:2.

2. Un gène selon la revendication 1, ledit gène provenant d'une espèce choisie du groupe comprenant l'humain, la souris, le rat et la levure.

3. Une protéine dont la séquence d'acides aminés consiste en la séquence décrite dans SEQ ID NO:2.

4. Une protéine selon la revendication 3, ladite protéine provenant d'une espèce choisie du groupe comprenant l'humain, la souris, le rat et la levure.

5. Un anticorps dirigé contre un gène selon la revendication 1.

6. Un anticorps dirigé contre une protéine selon la revendication 3.


### SEQUENCE LISTING

<110> ALAOUI-JAMALI, Moulay, A.
   CHO, John, M.-J.
<120> REPLICATION PROTEIN A BINDING
   TRANSCRIPTIONAL FACTOR (RBT1) AND USES THEREOF
<130> 14226-4PCT
<150> US 60/149,472
   <151> 1999-08-19
<160> 2
<170> FastSEQ for Windows Version 3.0
<210> 1
   <211> 591
   <212> DNA
   <213> unknown
<220>
   <223> replication protein A transcriptional factor
<400> 1
   atggagggag gcttgaagag gaaacactct gatttggaag aggaggagga gaggtgggag      60
   tggagtccag caggccttca gagctaccag caagccctgc tccgcatctc cctagacaaa     120
   gtccagcgca gcctgggccc ccgagcaccc agcctccgca ggcatgtcct catccataac     180
   accctccaac agctgcaggc tgcacttcgc ctggctcccg cccctgccct gccccccgag     240
   cccctcttcc tgggcgagga ggatttctcc ctgtcagcca ccattggctc tatcctcagg     300
   gagctggaca cctccatgga tgggactgag ccccctcaga atccagtgac tccccttggc     360
   ctccagaatg aagtgccacc ccagcctgat ccagtcttct tagaagctct gagctcccgg     420
   tacttggggg actctggcct ggatgacttc tttctggaca ttgacacatc tgcggtagaa     480
   aaggagcctg cacgggcccc accagagcct cctcacaacc tcttctgtgc cccaggttct     540
   tgggagtgga atgaactgga tcacatcatg gaaatcattc tggggtccta a              591
<210> 2
   <211> 196
   <212> PRT
   <213> unknown
<220>
   <223> replication protein A transcriptional factor
<400> 2